# EUROPEAN PATENT APPLICATION

(11) **EP 4 083 187 A1**
(43) Date of publication of application: **02.11.2022**
(21) Application number: 20907502.7
(22) Date of filing: 24.12.2020
(51) Int. Cl.: C12N 1/20, A23C 9/12

(54) **LACTIC ACID BACTERIUM FERMENTATION PROMOTER**

(30) Priority: 27.12.2019 JP 2019239455
(71) Applicant: MEIJI CO., LTD, Chuo-ku Tokyo 104-8306 (JP)
(72) Inventor: YAMAMOTO Eri, Hachioji-shi, Tokyo 192-0919 (JP); TSUCHIYA Asami, Hachioji-shi, Tokyo 192-0919 (JP); WATANABE Reiko, Hachioji-shi, Tokyo 192-0919 (JP); FUJIWARA Kayo, Hachioji-shi, Tokyo 192-0919 (JP)
(74) Representative: TBK
(86) International application number: PCT/JP2020/048328
(87) International publication number: WO 2021/132418

(57) **Abstract**

The present invention relates to a lactic acid bacterial fermentation promoter, containing at least one organic acid selected from malic acid and fumaric acid.

## Description

### Cross Reference to Related Applications

The present patent application claims priority based on Japanese Patent Application No. 2019-239455 filed on December 27, 2019, and the whole disclosures of the earlier patent applications are hereby incorporated by reference as a part of this description.

### BACKGROUND OF THE INVENTION

### Field of the Invention

The present invention relates to a novel lactic acid bacterial fermentation promoter.

### Background Art

In the industrial production of fermentates and metabolites by microorganisms, shortening the fermentation time is important for reduction of manufacturing costs and hygiene management. As a technique for shortening the fermentation time by promoting lactic acid bacterial fermentation, it has been reported that a substance of a raw material for nucleic acid promotes lactic acid bacterial fermentation (PTL 1). Further, PTL 2 describes a microbial production improver which contains an active ingredient, such as organic acid extract from Brassicaceae plant seeds. However, from the viewpoint of producing fermented milk efficiently on an industrial scale, there is still required a new technical means for easily promoting the metabolism and fermentation action of lactic acid bacteria.

On the other hand, the increasing health consciousness in recent years prompts request to reduce the amount of food additives used in fermented milk for a good taste. In particular, succinic acid is often used as a food additive because it has useful physiological functions such as suppressing weight gain, improving glucose tolerance, and suppressing cancer growth as well as being an umami substance. From the viewpoint of stimulating consumer demand, it is also preferable that succinic acid is produced in fermented milk without using food additives.

In recent years, a report has been written regarding the production mechanism of succinic acid: oxaloacetate is produced from pyruvic acid or phosphoenolpyruvic acid via carbon dioxide fixation under anaerobic conditions, and succinic acid may be produced through the reverse route of TCA cycle (NPL 1). However, there is no report that lactic acid bacteria can utilize the reverse route of the TCA cycle.

### Prior Art Documents

### Non Patent Documents

[NPL 1] Tatsuo Hoshino, Microbiol. Cult. Coll., 2011, 27 (2), p. 83-88

### Patent Documents

[PTL 1] Japanese Unexamined Patent Application Publication No. 2017-221157
[PTL 2] Japanese Unexamined Patent Application Publication No. 04-141083

### SUMMARY OF THE INVENTION

The present invention provides a new technical means for promoting lactic acid bacterial fermentation. Further, the present invention also provides a novel technical means for promoting the production of succinic acid by lactic acid bacteria in fermented milk.

The present inventors have now found that at least one organic acid selected from malic acid and fumaric acid can promote lactic acid bacterial fermentation. Further, the present inventors have found that the organic acid can promote the production of succinic acid by lactic acid bacteria in fermented milk. The present invention is based on such findings.

According to the present invention, the following inventions are included.
[1] A lactic acid bacterial fermentation promoter, comprising at least one organic acid selected from malic acid and fumaric acid.
[2] The fermentation promoter according to [1], wherein the fermentation promoter is for use in combination with a nucleic acid raw material.
[3] The fermentation promoter according to [2], wherein the nucleic acid raw material is at least one selected from the group consisting of formic acid and a compound having a purine skeleton with a hydrogen atom attached to a carbon atom at 2-position.
[4] The fermentation promoter according to any one of [1] to [3], wherein the lactic acid bacteria comprises genus Lactobacillus.
[5] The fermentation promoter according to [4], wherein the genus Lactobacillus is at least one selected from the group consisting of Lactobacillus delbrueckii, Lactobacillus delbrueckii subsp. bulgaricus, Lactobacillus acidophilus, Lactobacillus gasseri, Lactobacillus rhamnosus, Lactobacillus reuteri, Lactobacillus salivarius, and Lactobacillus pentosus.
[6] A lactic acid bacterial starter comprising lactic acid bacteria and at least one organic acid selected from malic acid and fumaric acid.
[7] The lactic acid bacterial starter according to [6], further comprising a nucleic acid raw material.
[8] A method for producing fermented milk, comprising: performing lactic acid bacterial fermentation in the presence of at least one organic acid selected from malic acid and fumaric acid.
[9] The method according to [8], further comprising performing lactic acid bacterial fermentation in the presence of the organic acid and a nucleic acid raw material.
[10] The method according to [9], wherein the nucleic acid raw material is at least one selected from formic acid and a compound having a purine skeleton with a hydrogen atom attached to a carbon atom at 2-position.
[11] The method according to any one of [8] to [10], wherein the fermented milk comprises succinic acid.
[12] The method according to [11], wherein the succinic acid is an endogenous organic acid produced by the lactic acid bacteria.
[13] A fermented milk obtained by the method according to any one of [8] to [12],
   the fermented milk comprising lactic acid bacteria and succinic acid.
[14] The fermented milk according to [13], wherein the succinic acid is an endogenous organic acid produced by the lactic acid bacteria.
[15] The fermented milk according to [14], wherein the content of the succinic acid is 0.15 mM or more with respect to the total amount of fermented milk.
[16] The fermented milk according to any one of [13] to [15], further comprising at least one organic acid selected from malic acid and fumaric acid.
[17] A method for promoting lactic acid bacterial fermentation, comprising: performing lactic acid bacterial fermentation in the presence of at least one organic acid selected from malic acid and fumaric acid.
[18] The method for promoting lactic acid bacterial fermentation according to [17], further comprising a nucleic acid law material.
[19] The method for promoting lactic acid bacterial fermentation according to [18], wherein the nucleic acid raw material is at least one selected from formic acid and a compound having a purine skeleton with a hydrogen atom attached to a carbon atom at 2-position.
[20] A method for producing a lactic acid bacterial starter, comprising: culturing lactic acid bacteria in the presence of at least one organic acid selected from malic acid and fumaric acid.
[21] The method according to [20], further comprising culturing lactic acid bacteria in the presence of the organic acid and a nucleic acid raw material.
[22] The method according to [20] or [21], wherein the lactic acid bacterial starter comprises lactic acid bacteria and at least one organic acid selected from malic acid and fumaric acid.
[23] The method according to [22], wherein the lactic acid bacterial starter further comprises succinic acid.
[24] The method according to [23], wherein the succinic acid is an endogenous organic acid produced by the lactic acid bacteria.
[25] A fermented milk having a succinic acid at a content of 0.15 mM or more with respect to the total amount of fermented milk.
[26] The fermented milk according to [25], further comprising at least one organic acid selected from malic acid and fumaric acid.
[27] The fermented milk according to [25] or [26], wherein the succinic acid is an endogenous organic acid produced by the lactic acid bacteria.
[28] A method for producing a lactic acid bacterial fermentation metabolite, comprising: performing lactic acid bacterial fermentation in the presence of at least one organic acid selected from malic acid and fumaric acid.
[29] The production method according to [28], wherein the lactic acid bacterial fermentation metabolite is an extracellular polysaccharide (EPS).
[30] A method for promoting production of lactic acid bacterial fermentation metabolite, comprising: performing lactic acid bacterial fermentation in the presence of at least one organic acid selected from malic acid and fumaric acid (hereinafter, also referred to as "production promoting method").
[31] The method according to [30], wherein the lactic acid bacterial fermentation metabolite is an extracellular polysaccharide (EPS).

According to the present invention, lactic acid bacterial fermentation can be promoted. Further, according to the present invention, the amount of succinic acid produced in fermented milk can be increased. The present invention can be advantageously utilized for promoting lactic acid bacterial fermentation and metabolism and producing fermentation metabolites such as extracellular polysaccharides (EPS) and peptides in a short time. Further, the present invention can be advantageously used for increasing the amount of succinic acid produced by lactic acid bacteria and thus reducing the amount of succinic acid added as a food additive.

### BRIEF DESCRIPTION OF THE DRAWINGS

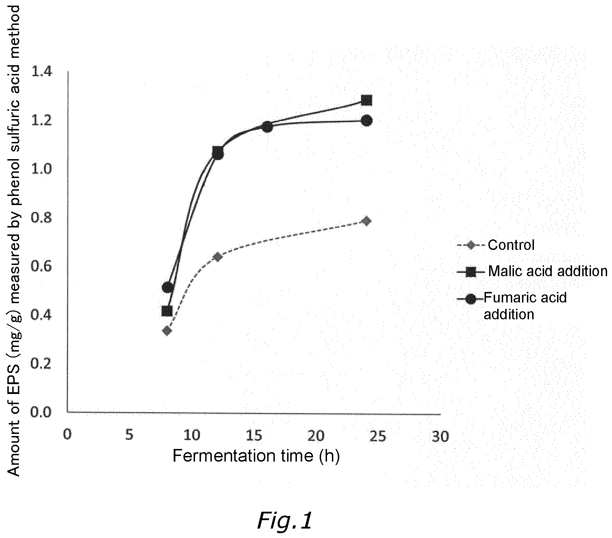

Fig. 1 is a chart showing change in EPS concentration with the culturing time of test example 9.

### DETAILED DESCRIPTION OF THE INVENTION

### Fermentation promoter

One of the characteristics of the lactic acid bacterial fermentation promoter of the present invention is containing at least one organic acid selected from malic acid and fumaric acid.

The fermentation promoter contains at least one organic acid selected from malic acid and fumaric acid, though the content is not particularly limited, for example, at a content of 0.1 to 100 mass%, preferably 50 to 100 mass%, and more preferably 80 to 100 mass%.

As a raw material for the organic acids in the fermentation promoter, there may be used a commercially available food additive, synthetic product, preparation containing an organic acid, or the like.

The fermentation promoter may contain an organic acid either of malic acid or fumaric acid, or may contain both. The mass ratio of malic acid to fumaric acid (malic acid/fumaric acid) is not particularly limited, but is, for example, 0.1 to 10, preferably 0.2 to 5, more preferably 0.5 to 2, and even more preferably 0.6 to 1.5.

### Malic acid

The malic acid in the fermentation promoter may be in any form as long as it does not hinder the effects of the present invention, and may be contained in the promoter in either form of free acid or salt. Examples of such salts include salts of alkali metal, such as potassium and sodium, and salts of alkaline earth metal, such as calcium and magnesium. The malic acid used for culturing may be of any optical isomer, but is preferably L-malic acid.

The malic acid or a salt thereof can be added in an amount, for example, in a range of 0.001 to 75 mM, preferably 0.01 to 50 mM, more preferably 0.1 to 10 mM, and still more preferably 0.5 to 10 mM, with respect to the total amount of the culture medium or raw material milk. Therefore, malic acid is preferably contained in the fermentation promoter in the above amount. Here, the total amount of the culture medium or raw material milk refers to the total amount of all the components used for culturing other than the bacteria, for example, the total amount of the medium or raw material milk, the malic acid and/or fumaric acid, and the nucleic acid raw material. The content of malic acid in the culture system of the present invention is measured by a high performance liquid chromatograph (HPLC) method. Such a measurement can be carried out by using a commercially available HPLC apparatus (for example, manufactured by Shimadzu Corporation) and a column (for example, ICSep ICE-ORH-801 (TRANSGENOMIC)). More specifically, the above measurement can be performed under the following conditions. Analytical instrument: LC20 system manufactured by Shimadzu Corporation, Column: ICSep ICE-ORH-801, 6.5 mm I.D. × 300 mm, used by connecting two, mobile phase: 7.5 mM p-toluenesulfonic acid, reaction solution: 7.5 mM p-toluenesulfonic acid/150 µM EDTA (2NA)/30 mM Bis Tris, flow rate: 0.5 mL/min, injection volume: 10 µL, oven temperature: 55 °C, detection: electric conductivity detector.

### Fumaric acid

The fumaric acid in the fermentation promoter may be in any form as long as it does not hinder the effect of the present invention, and may be contained in the promoter in either form of free acid or salt. Examples of such salts include salts of alkali metal, such as potassium and sodium, salts of alkaline earth metal, such as calcium, and ammonium salts.

Further, the fumaric acid or a salt thereof can be added in an amount, for example, in a range of 0.001 to 10 mM, preferably 0.01 to 7.5 mM, more preferably 0.1 to 5 mM, and still more preferably 0.1 to 2.5 mM, with respect to the total amount of the culture medium or raw material milk. Therefore, fumaric acid is preferably contained in the fermentation promoter of the present invention in the above amount. The content of fumaric acid in the culture system of the present invention can be measured by the same method as malic acid.

### Nucleic acid raw material

The fermentation promoter of the present invention is preferably used in combination with a nucleic acid raw material from the viewpoint of more effective promotion of the lactic acid bacterial fermentation. The nucleic acid raw material may be contained as a constituent component of the fermentation promoter or may be used as a separate substance. The nucleic acid raw material is not particularly limited as long as it does not hinder the effect of the present invention, and preferred examples thereof include formic acid and a compound having a purine skeleton with a hydrogen atom attached to a carbon atom at 2-position.

Formic acid is known as a raw material constituting the purine skeleton of nucleic acid. The formic acid may be in any form as long as it does not hinder the effects of the present invention, and may be contained in the promoter in either form of free acid or salt. Examples of such salts include salts of alkali metal, such as potassium and sodium, salts of alkaline earth metal, such as calcium, and ammonium salts. The content of formic acid in the culture system of the present invention can be measured by the same method as malic acid.

A compound having a purine skeleton refers to a substance having the following structure (purine skeleton) as a basic skeleton. 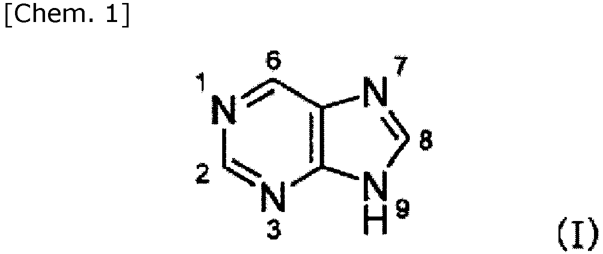 [The numbers in formula (I) represent the position numbers of carbon or nitrogen atoms]

A compound having a purine skeleton is also generally referred to as purine body. Examples of the compound having a purine skeleton typically include purine base, purine nucleoside, purine nucleotide, or a salt thereof.

The compound having a purine skeleton used as a nucleic acid raw material of the present invention has a purine skeleton with a hydrogen atom attached to a carbon atom at 2-position of the purine skeleton (a carbon atom represented by 2 in the above formula (I)). Examples of such a compound include adenine and hypoxanthine (purine base), purine nucleoside containing adenine and hypoxanthine as components, purine nucleotide containing adenine or hypoxanthine as components, and salts thereof.

The purine nucleoside is a substance consisting of a purine base attached to a sugar (ribose, deoxyribose, or the like), and may be a ribonucleoside or a deoxyribonucleoside. Examples of purine nucleosides containing adenine or hypoxanthine as components include adenosine and inosine (ribonucleoside), and deoxyadenosine and deoxyinosine (deoxyribonucleoside).

The purine nucleotide is a substance consisting of a purine nucleoside attached to one or more phosphate groups, and may be a ribonucleotide or a deoxyribonucleotide. The purine nucleotide may be nucleoside monophosphate, nucleoside diphosphate, or nucleoside triphosphate. Examples of the purine nucleotide containing adenine or hypoxanthine as a component include adenylic acid (adenosine monophosphate; AMP), adenosine diphosphate (ADP), adenosine triphosphate (ATP), deoxyadenosine monophosphate (dAMP), deoxyadenosine diphosphate (dADP), deoxyadenosine triphosphate (dATP), inosinic acid (inosine monophosphate; IMP), inosine diphosphate (IDP), inosine triphosphate (ITP), deoxyinosine monophosphate (dIMP), deoxyinosine diphosphate (dIDP), and deoxyinosine triphosphate (dITP).

The compound having a purine skeleton with a hydrogen atom attached to a carbon atom at 2-position also includes a derivative of a purine base, a purine nucleoside, or a purine nucleotide. In the present invention, the "derivative" refers to a compound in which a purine base, or a purine base portion, a sugar residue portion, and/or a phosphate group portion of a purine nucleoside or a purine nucleotide is chemically modified or substituted with a substituent.

The compound having a purine skeleton with a hydrogen atom attached to a carbon atom at 2-position may be a salt, for example, a salt of adenine or hypoxanthine, or of a purine nucleoside or a purine nucleotide containing adenine or hypoxanthine as a component. In the present invention, the preferred salts are alkali metal salts (for example, sodium salts, potassium salts), such as sodium adenylate and sodium inosinate, but not limited thereto.

The compound having a purine skeleton with a hydrogen atom attached to a carbon atom at 2-position is selected from the group consisting of, for example, adenine, hypoxanthine, adenosine, inosine, deoxyadenosine, deoxyinosine, adenylic acid, inosinic acid, and salts thereof, and is preferably inosinic acid. The content of inosinic acid in the culture system of the present invention can be measured using a kit by a fluorescence method, Inosine Assay Kit manufactured by Cell Biolabs, Inc.

The fermentation promoter of the present invention may use at least one kind of compound having a purine skeleton with a hydrogen atom attached to the carbon atom at 2-position, and may use preferably 1 to 4 kinds in combination, for example, 1 to 3 kinds or 1 to 2 kinds.

When the nucleic acid raw material is used in combination with an organic acid, the mass ratio of the organic acid to the nucleic acid raw material (organic acid/nucleic acid raw material) is not particularly limited, and is, for example, 0.005 to 500, preferably 0.05 to 200, and more preferably 0.1 to 100.

Further, the nucleic acid raw material can be added in an amount, for example, in a range of 0.001 to 75 mM, preferably 0.01 to 50 mM, and more preferably 0.1 to 10 mM, still more preferably 0.5 to 2 mM, with respect to the total amount of the culture medium or raw material milk.

In addition, the agent of the present invention can be provided as an agent containing a food hygienically and pharmaceutically acceptable additive, as desired, as well as the above-mentioned components. The food hygienically or pharmaceutically acceptable additives include aqueous media such as water, solvent, solubilizer, lubricant, emulsifier, tonicity agent, stabilizer, preservative, antiseptic, surfactant, regulator, chelating agent, pH regulator, buffer, excipient, thickener, colorant, aromatic or fragrance, and the like.

The agent of the present invention may be in any form such as liquid, powder, granule, gel, solid, capsule inclusion body, and the like.

The fermentation promoter can be produced by appropriately mixing at least one organic acid selected from malic acid and fumaric acid with other optional components such as nucleic acid, and the resulting mixture can be further processed according to known formulation techniques by dissolving in a solvent, powdering, granulation, gelation, solidification, encapsulation, and the like.

The fermentation promoting action of the present invention can be confirmed as follows: culturing lactic acid bacteria in raw material milk to which a fermentation promoter has been added, fermenting the raw material milk, examining an index indicating the progress of fermentation state, and comparing the result with a control (a group without adding the fermentation promoter of the present invention) to judge that the fermentation is proceeding faster than the control. The index indicating the progress of the fermentation state is not particularly limited, but for example, a decrease in the pH value of the fermented milk due to an increase in the amount of lactic acid produced by lactic acid bacterial fermentation can be used as an index. The pH value is, for example, pH 4.6. Here, pH 4.6 can be used as a pH setpoint which indicates the completion of fermentation of sufficiently fermented state in the production of normal fermented milk. When the time to reach pH 4.6 is reduced as compared with the control, the fermentation promoter can be determined to have a fermentation promoting action on lactic acid bacteria. The pH value can be measured using a commercially available pH meter.

### Lactic acid bacteria

In the present invention, the lactic acid bacteria used for fermentation is not particularly limited as long as it does not hinder the effect of the present invention, and may be of animal origin or plant origin.

Examples of preferred lactic acid bacteria of the present invention include Lactobacillus spp., Streptococcus spp., Lactococcus spp., Enterococcus spp., Leuconostoc spp., and combinations thereof, and lactic acid bacteria containing Lactobacillus spp. is preferable. Examples of the lactic acid bacteria containing Lactobacillus spp. include Lactobacillus spp., a combination of Lactobacillus spp. and Streptococcus spp., and a combination of Lactobacillus spp. and Lactococcus spp. These lactic acid bacteria can be obtained from, for example, a culture collection such as ATCC, or commercially available ones, and used as appropriate.

Examples of Lactobacillus spp. include Lactobacillus delbrueckii, Lactobacillus acidophilus, Lactobacillus gasseri, Lactobacillus rhamnosus, Lactobacillus reuteri, Lactobacillus salivarius, Lactobacillus pentosus, Lactobacillus kefiranofaciens, Lactobacillus helveticus, Lactobacillus johnsonii, Lactobacillus casei, Lactobacillus fermentum, Lactobacillus amylovorous, Lactobacillus brevis, Lactobacillus plantarum, Lactobacillus sakei and the like. Preferred examples of Lactobacillus spp. include Lactobacillus delbrueckii, Lactobacillus acidophilus, Lactobacillus gasseri, Lactobacillus rhamnosus, Lactobacillus reuteri, Lactobacillus salivarius, Lactobacillus pentosus and the like. Here, examples of preferred Lactobacillus acidophilus, Lactobacillus gasseri, Lactobacillus rhamnosus, Lactobacillus reuteri, Lactobacillus salivarius, Lactobacillus pentosus include Lactobacillus acidophilus JCM 1132T, Lactobacillus gasseri JCM 1131T, Lactobacillus rhamnosus JCM 1136T, Lactobacillus reuteri JCM 1112T, Lactobacillus salivarius JCM 1231T, and Lactobacillus pentosus JCM 1558T, respectively.

Further, examples of Lactobacillus delbrueckii include Lactobacillus delbrueckii subsp. bulgaricus (Lactobacillus delbrueckii subsp. bulgaricus; Lactobacillus bulgaricus), Lactobacillus delbrueckii subsp. lactis, Lactobacillus delbrueckii subsp. delbrueckii, Lactobacillus delbrueckii subsp. indicus and the like. Preferred examples of Lactobacillus delbrueckii include Lactobacillus delbrueckii subsp. bulgaricus and the like, and more preferred examples include Lactobacillus delbrueckii subsp. bulgaricus (2038, OLL 1073R-1, P1902901, OLL1171, OLL1255, OLL1247, OLL205013) and the like. Examples of Lactobacillus kefiranofaciens include Lactobacillus kefiranofaciens subsp. kefirgranum, and the like, and preferred examples include Lactobacillus kefiranofaciens subsp. kefirgranum JCM 8572T and the like.

In addition, Lactobacillus acidophilus JCM 1132T, Lactobacillus gasseri JCM 1131T, Lactobacillus rhamnosus JCM 1136T, Lactobacillus reuteri JCM 1112T, Lactobacillus salivarius JCM 1231T, Lactobacillus pentosus JCM 1558T, and Lactobacillus kefiranofaciens subsp. kefirgranum JCM 8572T can be obtained from Japan Collection of Microorganisms, BioResource Center, Incorporated Administrative Agency RIKEN (RIKEN BRC-JCM, Japan) under the accession numbers of JCM 1132T, JCM 1131T, JCM 1136T, JCM 1112T, JCM 1231T, JCM 1558T, and JCM 8572T, respectively.

Lactobacillus delbrueckii subsp. bulgaricus 2038 can be isolated from "Meiji Bulgaria yogurt" (registered trademark) on a commercially available selective medium for the genus Lactobacillus, and is stored by Meiji Co., Ltd. (Meiji Innovation Center, 1-29-1, Nanakuni, Hachioji-shi, Tokyo, 192-0919, Japan).

Lactobacillus delbrueckii subsp. bulgaricus OLL 1073R-1 was deposited on February 22, 1999 at the International Patent Organism Depositary, National Institute of Advanced Industrial Science and Technology (AIST Tsukuba Central 6, 1-1, Higashi, 1-chome Tsukuba-shi, Ibaraki-ken, Japan) and then converted to an international deposit and given the accession number FERM BP-10741. In addition, as described in Budapest Notification No. 282 (http://www.wipo.int/treaties/en/notifications/budapest/treaty_bud apest_282.html), since the National Institute of Technology and Evaluation (IPOD, NITE) succeeded the patented microorganism deposit business from the International Patent Organism Depositary (IPOD, AIST), it was deposited under the accession number FERM BP-10741 to the NITE Patent Organism Depositary, National Institute of Technology and Evaluation (IPOD, NITE).

Lactobacillus delbrueckii subsp. bulgaricus P1902901 is stored by Meiji Co., Ltd. (Meiji Innovation Center, 1-29-1, Nanakuni, Hachioji-shi, Tokyo, 192-0919, Japan).

Lactobacillus delbrueckii subsp. bulgaricus OLL1171 was internationally deposited on March 13, 2013 at the NITE Patent Microorganisms Depositary, National Institute of Technology and Evaluation, International Depositary Authority based on Budapest Treaty, under the accession number of NITE BP-01569.

Lactobacillus delbrueckii subsp. bulgaricus OLL1255 was internationally deposited on February 10, 2005 at the NITE Patent Microorganisms Depositary, National Institute of Technology and Evaluation, International Depositary Authority based on Budapest Treaty, under the accession number of NITE BP-76.

Lactobacillus delbrueckii subsp. bulgaricus OLL1247 was internationally deposited on March 6, 2014 at the NITE Patent Microorganisms Depositary, National Institute of Technology and Evaluation, International Depositary Authority based on Budapest Treaty, under the accession number of NITE BP-01814.

Lactobacillus delbrueckii subsp. bulgaricus OLL205013 was internationally deposited on February 3, 2017 at the NITE Patent Microorganisms Depositary, National Institute of Technology and Evaluation, International Depositary Authority based on Budapest Treaty, under the accession number of NITE BP-02411.

Examples of the Streptococcus spp. include Streptococcus thermophilus and the like.

Examples of the Lactococcus spp. include Lactococcus lactis, Lactococcus plantarum, Lactococcus raffinolactis and the like.

Examples of the combination of Lactobacillus spp. and Streptococcus spp. preferably include that of Lactobacillus delbrueckii subsp. bulgaricus and Streptococcus thermophilus.

The mixing ratio of the lactic acid bacteria, at least one organic acid selected from malic acid and fumaric acid, and the nucleic acid raw material may be appropriately set depending on type and property of lactic acid bacteria, culture medium, and raw material milk, and fermentation conditions such as temperature and the like.

The mass ratio of the lactic acid bacteria to at least one organic acid selected from malic acid and fumaric acid (lactic acid bacteria/organic acid) is, for example, 0.001 to 500000, preferably 0.01 to 5000, and more preferably 0.1 to 500.

The mass ratio of the lactic acid bacteria to the nucleic acid raw material (lactic acid bacteria/nucleic acid raw material) is, for example, 0.1 to 11000, preferably 1 to 11000, more preferably 10 to 1100, and further preferably 50 to 550.

The lactic acid bacteria can be added in an amount of, for example, 0.001 to 5 mass%, preferably 0.01 to 2.5 mass%, more preferably 0.01 to 2 mass%, and still more preferably 0.1 to 1 mass% with respect to the total amount of the culture medium or raw material milk.

### Lactic acid bacterial starter

The organic acid selected from malic acid and fumaric acid as described above can be used as a lactic acid bacterial starter together with lactic acid bacteria. Therefore, according to a preferred embodiment of the present invention, there is provided a lactic acid bacterial starter containing lactic acid bacteria and at least one organic acid selected from malic acid and fumaric acid.

The lactic acid bacterial starter includes a product prepared through intermediate fermentation by culturing lactic acid bacteria in a culture medium (for example, an activation medium). The lactic acid bacterial starter preferably contains as its components lactic acid bacteria and a medium in which the lactic acid bacteria are cultured. Therefore, the lactic acid bacterial starter may further contain succinic acid. Here, the succinic acid is preferably an endogenous organic acid produced by the lactic acid bacteria. The lactic acid bacterial starter includes those of next or further generation obtained by inoculating a lactic acid bacterial starter into another medium and further growing (scale up), as well as those obtained by inoculating lactic acid bacteria directly into raw material milk used for the fermented milk.

The lactic acid bacterial starter is basically used to ferment raw material milk to obtain fermented milk. Use of the lactic acid bacterial starter also includes inoculating raw material milk with a lactic acid bacterial starter of next or further generation which is prepared by culturing once or more a lactic acid bacterial starter obtained by the present invention in a medium, as well as inoculating directly raw material milk with a lactic acid bacterial starter obtained by the present invention.

The lactic acid bacterial starter may be used in combination with the nucleic acid raw material. Therefore, according to another aspect of the present invention, there is provided a lactic acid bacterial starter, containing lactic acid bacteria and at least one organic acid selected from malic acid and fumaric acid, for use in combination with a nucleic acid raw material. The lactic acid bacterial starter is preferably used in combination with the nucleic acid raw material, wherein the nucleic acid raw material may be added as a separate substance together with the lactic acid bacterial starter to the culture medium or raw material milk, or the nucleic acid raw material may be mixed as a constituent component with the lactic acid bacterial starter to be used integrally. Use of the lactic acid bacterial starter in combination with the nucleic acid raw material can promote the fermentation of the lactic acid bacteria more effectively. Therefore, according to one embodiment, the lactic acid bacterial starter may contain a nucleic acid raw material. Each embodiment of malic acid, fumaric acid, nucleic acid raw material, and lactic acid bacteria in the lactic acid bacterial starter can be the same as in the description regarding the fermentation promoter of the present invention.

The viable cell count of lactic acid bacteria in the lactic acid bacterial starter is not particularly limited, but is, for example, 1.0 × 10⁴ to 1.0 × 10¹³ cfu/g, preferably 1.0 × 10⁵ to 1.0 × 10¹² cfu/g, and more preferably 1.0 × 10⁶ to 1.0 × 10¹¹ cfu/g.

The mass ratios in the lactic acid bacterial starter such as lactic acid bacteria to an organic acid, lactic acid bacteria to a nucleic acid raw material, an organic acid to a nucleic acid raw material, and malic acid to fumaric acid in the organic acid can be the same as the mass ratios in the above fermentation promoter.

The lactic acid bacterial starter can be produced from the lactic acid bacteria and the above-mentioned optional components such as an organic acid, a nucleic acid raw material, and a medium component. Hereinafter, details of a suitable method for producing a lactic acid bacterial starter will be described.

A preferred method for producing a lactic acid bacterial starter includes steps of preparing medium, sterilizing a medium, inoculating lactic acid bacteria, culturing (medium fermentation), and adding an organic acid and the like.

The step of preparing a medium is a step of preparing a medium (for example, an activation medium) to be inoculated with lactic acid bacteria. The medium is not particularly limited as long as it does not hinder the effect of the present invention, and includes a medium containing milk constituents: preferably a medium containing, for example, skim milk, skim milk concentrate, skim milk powder (reduced skim milk), and protein decomposition products of these skim milk components; whey, whey concentrate, whey powder (reduced whey), and protein decomposition products of these whey components; raw milk, sterilized milk (full fat milk), concentrated full fat milk, full fat milk powder (reduced full fat milk), and protein decomposition products of these full fat milk components; and the like; more preferably a medium containing skim milk, skim milk powder, and protein decomposition products of these skim milk components; whey, whey concentrate, whey powder, and protein decomposition products of these whey components; and further preferably a medium containing, for example, skim milk, skim milk powder, whey, whey powder, and the like. Further, the above-mentioned medium may be the same as the raw material milk described later. The above-mentioned medium preferably further contains yeast extract. The above-mentioned medium can be prepared from each of the above components by a known method such as mixing, dissolving, dispersing, suspending, and the like.

The step of sterilizing a medium is a step of sterilizing the medium prepared in the step of preparing a medium by, for example, heating. In the sterilizing step, the heat treatment should be performed by adjusting the heating temperature and heating time to kill germs in the medium. In the present invention, the medium is heated preferably to 80°C or higher, 90°C or higher, 95°C or higher, or 100°C or higher. The heat sterilization can adopt known methods. For example, heat sterilization may be performed by heat treatment using a plate type heat exchanger, a tube type heat exchanger, a steam injection type heating device, a steam infusion type heating device, an electric heating device, an autoclave device, and the like, or by a jacketed tank. The sterilization of the medium is not limited to heating, and can also be performed by a known method such as ultraviolet irradiation.

The step of adding lactic acid bacteria (inoculating) is a step of adding (inoculating) lactic acid bacteria to the sterilized medium. Frozen bacteria (for example, freeze-concentrated bacteria, frozen pellets, freeze-dried powder, and the like) can be used for adding to the medium. In the step of adding lactic acid bacteria, lactic acid bacteria are added preferably in an amount of 0.05 mass% or more, more preferably 0.05 to 10 mass%, and further preferably 0.1 to 5 mass% with respect to the medium.

The culturing step is a step of culturing lactic acid bacteria in a medium, growing the lactic acid bacteria, and obtaining a lactic acid starter. The culturing time of the lactic acid bacteria is not particularly limited, but is, for example, 3 to 36 hours, preferably 5 to 30 hours, and more preferably 10 to 24 hours. When the lactic acid bacterial starter is obtained by a plurality of culturing times, the above-mentioned culturing time means one cycle of culturing time.

Further, in the culturing step, the temperature of the medium is preferably maintained in the fermentation temperature range of 30°C or higher. In particular, the temperature of the medium is preferably maintained at 30 to 50°C and more preferably 35 to 50°C. Further, in the culturing step, the medium is preferably left to stand without stirring. Here, "stand" means that the medium is not stirred, and, for example, "stand" is applicable to the case in which the container containing the medium is moved but the inside of the medium is not stirred. In this way, leaving the medium to stand during the culturing step promote the growth of lactic acid bacteria, which can reduce the time until the completion of culture.

The step of adding an organic acid and the like is a step of adding at least one organic acid selected from malic acid and fumaric acid, and, if desired, a nucleic acid raw material. The step of adding an organic acid and the like may be carried out at any time such as before culturing step, during culturing step, and after culturing step, but is preferably carried out before culturing step or during culturing step, from the viewpoint of reducing the culturing time. The step of adding an organic acid and the like can be carried out by, for example, after the culturing step, taking a predetermined amount of lactic acid bacteria or a medium containing the lactic acid bacteria, and adding the organic acid and, if desired, a nucleic acid raw material thereto. Further, the step of adding an organic acid and the like may be carried out by adding an organic acid and, if desired, a nucleic acid raw material to the medium, for example, before or during the culturing step. Here, the timing for the step of adding an organic acid and the like is not particularly limited before the culturing step, and may be performed at any time: before the step of sterilizing a medium, after the step of sterilizing a medium, before the step of adding lactic acid bacteria, after the step of adding lactic acid bacteria, and together with adding lactic acid bacteria. The organic acid to be added is preferably dissolved in water and adjusted to pH 6.0 to 7.0.

### Fermented milk

According to the present invention, fermented milk can be efficiently produced by using the above-mentioned fermentation promoter or lactic acid bacterial starter. Here, "fermented milk" includes "fermented milk", "dairy product lactic acid bacteria beverage", and "lactic acid bacteria beverage" defined by the ministerial ordinance concerning compositional standards, etc. for milk and milk products (Ministerial Ordinance on Milk, etc.), and yogurt, etc. Fermented milk refers to those obtained by fermenting the following kinds of milk with lactic acid bacteria or yeast to form a paste or liquid, or the frozen thereof, which includes hard yogurt, soft yogurt (paste-like fermented milk), or drink yogurt (liquid fermented milk), wherein the kinds of milk include raw milk, cow milk, certified cow milk, raw goat milk, sterilized goat milk, raw sheep milk, ingredient-adjusted milk, lowfat milk, non-fat milk and processed milk, or milk containing non-fat milk solids at an amount equal to or higher than them.

Generally, hard yogurt such as plain yogurt is produced by filling a container with a raw material and then fermenting it (postfermentation) (also referred to as "set yogurt"). On the other hand, soft yogurt and drink yogurt are produced by atomizing or homogenizing fermented milk (pre-fermented) and then filling it in a container (also referred to as "stirred yogurt").

According to the present invention, performing lactic acid bacterial fermentation in the presence of malic acid, fumaric acid, and further, if desired, nucleic acid raw materials can allow a high content of succinic acid to be contained in fermented milk without using food additives. Therefore, according to a preferred embodiment of the present invention, there is provided a fermented milk containing lactic acid bacteria and succinic acid, wherein the succinic acid is an endogenous organic acid produced by the lactic acid bacteria.

The content of succinic acid in the fermented milk is, for example, 0.15 mM or more, preferably 0.2 mM or more, more preferably 0.7 mM or more, still more preferably 1 mM or more, and further preferably 3 mM or more with respect to the total amount of fermented milk. The preferred lower limit of the succinic acid content in the fermented milk of the present invention is 0.15 mM, preferably 1 mM, and more preferably 3 mM, and the preferred upper limit is 50 mM, more preferably 20 mM, and even more preferably 15 mM. According to a preferred embodiment of the present invention, when Lactobacillus delbrueckii subsp. bulgaricus is used as the lactic acid bacteria, the succinic acid content in the fermented milk is, for example, 1 mM or more, preferably 1.5 mM or more, and more preferably 3 mM or more with respect to the total amount of fermented milk. Further, when Lactobacillus rhamnosus is used as the lactic acid bacteria, the succinic acid content in the fermented milk is, for example, 0.5 mM or more, and preferably 1 mM or more with respect to the total amount of fermented milk. Further, when Lactobacillus salivarius is used as the lactic acid bacteria, the succinic acid content in the fermented milk is, for example, 0.15 mM or more, and preferably 0.2 mM or more with respect to the total amount of fermented milk. The content of succinic acid in the fermented milk of the present invention is measured by a high performance liquid chromatograph (HPLC) method. Such measurement can be easily performed by using a commercially available HPLC apparatus (for example, manufactured by Shimadzu Corporation) and a column (for example, ICSep ICE-ORH-801 (TRANSGENOMIC)). The measurement can be easily performed, for example, under the following conditions. Analytical instrument: LC20 system manufactured by Shimadzu Corporation, Column: ICSep ICE-ORH-801, 6.5 mm I.D. × 300 mm, used by connecting two, mobile phase: 7.5 mM p-toluenesulfonic acid, reaction solution: 7.5 mM p-toluenesulfonic acid/150 µM EDTA (2NA)/30 mM Bis Tris, flow rate: 0.5 mL/min, injection volume: 10 µL, oven temperature: 55 °C, detection: electric conductivity detector.

Since the fermented milk is suitably produced in the presence of an organic acid such as malic acid and fumaric acid, and if desired, a nucleic acid raw material, the fermented milk may contain malic acid, fumaric acid, and a nucleic acid raw material.

The content of malic acid in the fermented milk is, for example, 0.1 to 50 mM, preferably 0.1 to 45 mM, and more preferably 0.5 to 45 mM.

The content of fumaric acid in the fermented milk is, for example, 0.1 to 10 mM, preferably 0.1 to 5 mM, and more preferably 0.5 to 1 mM.

The content of nucleic acid raw material in the fermented milk is, for example, 0.0001 to 5 mass% and preferably 0.0001 to 1.5 mass%.

The fermented milk of the present invention, comparing with a fermented milk to which at least one organic acid selected from malic acid and fumaric acid is not added at the time of production, is cited as containing twice or more succinic acid, and preferably contains 2.5 times or more, and more preferably 5 times or more. The upper limit is cited as 30 times and is preferably 20 times.

According to the present invention, fermented milk can be produced in the presence of organic acids such as malic acid and fumaric acid, and nucleic acid raw materials by using the above-mentioned fermentation promoter or lactic acid bacterial starter. Accordingly, there is provided a method for producing fermented milk, comprising fermenting raw material milk with lactic acid bacteria in the presence of at least one organic acid selected from malic acid and fumaric acid. Further, according to a preferred embodiment, the method for producing fermented milk is to perform lactic acid bacterial fermentation in the presence of the organic acid and the nucleic acid raw material.

More specifically, the method for producing fermented milk preferably includes a step of preparing raw material milk, a step of sterilizing raw material milk, a step of inoculating lactic acid bacterial starter, and a step of fermenting.

The step of preparing raw material milk is a step of preparing raw material milk to be inoculated with a lactic acid bacterial starter. "Raw material milk" is a raw material for fermented milk such as yogurt, which is also called yogurt mix or fermented milk mix. In the present invention, known raw material milk can be appropriately used. Raw material milk includes both pre-sterilized and post-sterilized.

Specific raw materials for raw material milk may include raw milk, sterilized milk, skim milk, full fat milk powder, skim milk powder, buttermilk, butter, cream, whey protein concentrate (WPC), whey protein isolate (WPI), alpha-lactoalbumin (La), betalactoglobulin (Lg) and the like. Pre-warmed gelatin or the like may be added as appropriate. Raw material milk is known and can be prepared according to known methods.

Preferred raw material milk includes, though not limited thereto, raw milk, skim milk, skim milk powder, and cream. More preferable raw material milk includes skim milk and skim milk powder. The content of non-fat milk solids in the raw material milk used in the present invention is not particularly limited as long as it does not hinder the effects of the present invention, and is preferably 6 to 11 mass% and more preferably 7 to 10 mass%. In addition, the content of fat in the raw material milk is not particularly limited as long as it does not hinder the effects of the present invention, and is exemplified as preferably 0.01 to 10 mass%, more preferably 0.05 to 5 mass%, and even more preferably 0.1 to 3 mass%, but is not limited thereto.

The step of sterilizing raw material milk is a step of sterilizing the raw material milk prepared in the step of preparing raw material milk by, for example, heating. Raw material milk is preferably sterilized. Examples of such sterilization include sterilization by, for example, heating. The heat treatment for sterilization can be performed by adjusting the heating temperature and the heating time so as to kill germs in the raw material milk. For example, the raw material milk is preferably sterilized at a temperature of 80°C or higher and preferably 90°C or higher. Known methods can be used for the heat treatment.

The step of inoculating a lactic acid bacterial starter is a step of inoculating (adding) the lactic acid bacterial starter to the above-mentioned raw material milk. As the above lactic acid bacterial starter, there can be used lactic acid bacterial starters: a lactic acid bacterial starter obtained through the above-mentioned method for producing the lactic acid bacterial starter, a lactic acid bacterial starter prepared by a usual method and frozen, or a lactic acid bacterial starter dried after freezing.

Here, at least one organic acid selected from malic acid and fumaric acid and nucleic acid raw material as optional component are preferably added to the raw material milk as a constituent component in the lactic acid bacterial starter, but may be contained separately from the lactic acid bacterial starter in raw material milk as an additive or as an endogenous component. The amount of each component of the lactic acid bacteria, the organic acid, and the nucleic acid raw material to be added to the raw material milk can be the same as the amount described in the above-mentioned fermentation promoter.

The step of fermenting is a step of fermenting raw material milk with a lactic acid bacterial starter. In the fermenting step, for example, fermented milk is obtained by fermenting the raw material milk inoculated with the lactic acid bacterial starter while maintaining the temperature in the fermentation temperature range. In the present invention, known methods can be used for the fermenting step. Fermentation conditions such as fermentation temperature can be appropriately adjusted in consideration of the types of raw material milk and lactic acid bacteria (lactic acid bacterial starter), the type and flavor of the fermented milk to be prepared, and the like. As a specific example, the fermentation temperature is cited as about 30 to 50°C. The temperature in this range can generally facilitate the action of lactic acid bacteria, and thus promote the fermentation effectively. The fermentation temperature at this time is preferably about 30 to 45°C and more preferably about 35 to 43°C.

The fermentation time may be appropriately adjusted depending on the lactic acid bacteria (lactic acid bacterial starter) used, the fermentation temperature, and the like. For example, the fermentation time can be adjusted by using an index of reaching pH 4.6 in the fermenting milk. The fermentation time is not limited, but may be, for example, 2 hours to 36 hours, preferably 2.5 hours to 24 hours, and more preferably 4 hours to 24 hours. The pH value can be measured using a commercially available pH meter.

In addition, as the apparatus for producing fermented milk and the production conditions, known one can be used. For example, as an apparatus for producing fermented milk, there can be used, for post-fermented product, a fermentation chamber for fermenting after filling, and, for pre-fermented product, a fermentation tank for fermenting and a line filter or homogenizer for crushing fermented milk curd, and for production conditions, there can be adopted a deoxidizing device or the like appropriately.

As described above, succinic acid contained in fermented milk is an organic acid having useful physiological functions such as suppressing weight gain, improving glucose tolerance, and suppressing cancer growth as well as being an umami substance. Therefore, according to one embodiment, the fermented milk of the present invention is provided as a composition for improving umami, suppressing weight gain, improving glucose tolerance, or suppressing cancer growth.

### Other embodiments

According to the present invention, as described above, lactic acid bacterial fermentation can be promoted by using at least one organic acid selected from malic acid and fumaric acid and, if desired, in combination with a nucleic acid raw material. Therefore, according to another embodiment of the present invention, there is provided a method for promoting lactic acid bacterial fermentation, comprising fermenting a medium (for example, a medium containing a milk constituent), raw material milk, or the like with lactic acid bacteria in the presence of at least one organic acid selected from malic acid and fumaric acid. Further, according to another preferred embodiment, the above-mentioned method for promoting lactic acid bacterial fermentation comprises performing lactic acid bacterial fermentation in the presence of the organic acid and a nucleic acid raw material.

According to the present invention, as described above, fermentation and metabolism with lactic acid bacteria can be promoted, and fermentation metabolites can be produced in a short time. Therefore, according to still another embodiment of the present invention, there is provided a method for producing or promoting the production of a lactic acid bacterial fermentation metabolite, comprising performing lactic acid bacterial fermentation of a medium (for example, a medium containing a milk constituent), raw material milk or the like in the presence of at least one organic acid selected from malic acid and fumaric acid. Further, according to another preferred embodiment, the method comprises performing lactic acid bacterial fermentation in the presence of the organic acid and the nucleic acid raw material. Examples of the fermentation metabolite include succinic acid, extracellular polysaccharide (EPS), peptide and the like, and more preferably extracellular polysaccharide (EPS).

Further, according to still another embodiment of the present invention, there is provided at least one organic acid selected from malic acid and fumaric acid for promoting lactic acid bacterial fermentation. Further, according to still another preferred embodiment, the organic acid enables lactic acid bacterial fermentation in the presence of a nucleic acid raw material.

Further, according to still another embodiment of the present invention, there is provided use of at least one organic acid selected from malic acid and fumaric acid in the production of a lactic acid bacterial fermentation promoter. Further, according to still another preferred embodiment, the organic acid is used in combination with a nucleic acid raw material.

Further, according to still another embodiment of the present invention, there is provided use of at least one organic acid selected from malic acid and fumaric acid in the production of a lactic acid bacterial starter. Further, according to still another preferred embodiment, the organic acid is used in combination with a nucleic acid raw material.

Further, according to still another embodiment of the present invention, there is provided use of at least one organic acid selected from malic acid and fumaric acid in the production of fermented milk, wherein the use includes performing lactic acid bacterial fermentation in the presence of the organic acid. Further, according to still another preferred embodiment, the organic acid is used in combination with a nucleic acid raw material. According to still another more preferred embodiment, the fermented milk contains succinic acid, which may be an endogenous organic acid produced by lactic acid bacteria.

Each embodiment of the above-mentioned method for promoting lactic acid bacterial fermentation, organic acid, and use can be carried out according to the description of the production method of the fermentation promoter, the lactic acid bacterial starter, and the fermented milk of the present invention.

### EXAMPLES

Hereinafter, the present invention will be described in more detail by way of examples, but the technical scope of the present invention is not limited to these examples. Unless otherwise specified, all ratios used in the present invention are based on mass. Unless otherwise specified, the units and measurement methods described in this specification are based on JIS standards.

### Test Example 1: Examination of fermentation promoting effect of malic acid or fumaric acid on Lactobacillus delbrueckii subsp. bulgaricus (hereinafter, also referred to as "L. bulgaricus")

First, frozen bacteria of the following Lactobacillus delbrueckii subsp. bulgaricus strains were prepared.
(1) Lactobacillus delbrueckii subsp. bulgaricus 2038 (hereinafter also referred to as "2038")
(2) Lactobacillus delbrueckii subsp. bulgaricus OLL1073R-1 (hereinafter, also referred to as "OLL 1073R-1")
(3) Lactobacillus delbrueckii subsp. bulgaricus P1902901 (hereinafter, also referred to as "P1902901")
(4) Lactobacillus delbrueckii subsp. bulgaricus OLL1171 (hereinafter, also referred to as "OLL1171")
(5) Lactobacillus delbrueckii subsp. bulgaricus OLL1255 (hereinafter, also referred to as "OLL1255")
(6) Lactobacillus delbrueckii subsp. bulgaricus OLL1247 (hereinafter, also referred to as "OLL1247")
(7) Lactobacillus delbrueckii subsp. bulgaricus OLL205013 (hereinafter, also referred to as "OLL205013")

The above strains were used after precultured in a preculture medium. The activation medium used was a 10 mass% reduced skim milk medium containing 0.1 mass% yeast extract, sterilized at 121°C for 7 minutes. Here, the 10 mass% reduced skim milk medium is a 10 mass% aqueous solution of skim milk powder (fat 1 mass%, protein 34 mass%, lactose 54 mass%, ash 8 mass%, non-fat milk solid 96 mass%) (manufactured by Meiji Co., Ltd.) (in the above medium, lactose 5.4 mass%, non-fat milk solid 9.6 mass%). Frozen bacteria were added to the above-mentioned activation medium in an amount of 0.1 mass% (with respect to the amount of the activation medium) and statically cultured at 37°C for 16 hours to obtain an activation solution. An aliquot of 0.1 mass% of the activation solution obtained (with respect to the amount of activation medium) was added to another activation medium, and the mixture was allowed to stand at 37°C for 16 hours to obtain a lactic acid bacterial starter.

Then, fermentation was carried out using a fermentation medium as raw material milk. The fermentation medium was prepared by adding formic acid to the 10 mass% reduced skim milk medium so as to give a final concentration of 1 mM, and then sterilizing the mixture at 95°C, and was used. Further, an aqueous fumaric acid solution or an aqueous malic acid solution, adjusted to a pH of about 6.5 using NaOH before the sterilization, was added to the fermentation medium so as to give a final concentration of 1 mM. The pH of the fermentation medium was about 6.4. The lactic acid bacterial starter of each strain obtained above was inoculated into the fermentation medium in an amount of 0.5 mass% (with respect to the amount of the fermentation medium), statically cultured at 40°C, and fermented. The fermentation time was the time required for the medium to reach pH 4.6. The pH value was measured using a commercially available pH meter. The results obtained are shown in Table 1. From Table 1, the addition of malic acid or fumaric acid is found to promote fermentation in all 7 L. bulgaricus strains. Malic acid and fumaric acid had almost the same fermentation promoting effects on L. bulgaricus. The reduction in fermentation time varied among strains and ranged from 40 minutes to 12 hours and 15 minutes.

### [Table 1]

**Table 1: Fermentation promoting effect of malic acid and fumaric acid on L. delbrueckii subsp. bulgaricus**

| | Fermentation time (Time to reach pH 4.6) | | | Reduction in fermentation time | |
|---|---|---|---|---|---|
| Strain | No addition | Malic acid (1 mM) added | Fumaric acid (1 mM) added | Malic acid (1 mM) added | Fumaric acid (1 mM) added |
| 2038 | 5:40 | 4:55 | 5:00 | 0:45 | 0:40 |
| OLL1073R-1 | 6:00 | 4:45 | 4:45 | 1:15 | 1:15 |
| P1902901 | 7:40 | 5:50 | 5:45 | 1:50 | 1:55 |
| OLL1171 | 10:05 | 6:25 | 6:25 | 3:40 | 3:40 |
| OLL1255 | 9:25 | 4:40 | 4:40 | 4:45 | 4:45 |
| OLL1247 | 10:35 | 4:20 | 4:20 | 6:15 | 6:15 |
| OLL205013 | 20:45 | 8:30 | 8:35 | 12:15 | 12:10 |

### Test Example 2-1: Examination of concentrations of malic acid and fumaric acid for exhibiting a fermentation promoting effect on L. bulgaricus 2038

The fermentation was carried out using a fermentation medium (raw material milk). The fermentation medium was prepared by adding formic acid to the 10% reduced skim milk medium so as to give a final concentration of 1 mM, and then sterilizing the mixture at 95°C, and was used. Further, an aqueous fumaric acid solution or an aqueous malic acid solution, adjusted to a pH of about 6.5 using NaOH before the sterilization, was added to the fermentation medium so as to have the final concentrations shown in Tables 3 and 4. In the above fermentation medium, a lactic acid bacterial starter of L. Lactobacillus 2038 was inoculated at 0.5% (with respect to the amount of fermentation medium), statically cultured at 40°C, and fermented. The fermentation time was the time required for the medium to reach pH 4.6.

The measurement of the organic acid concentration after the completion of fermentation was carried out as follows. An aliquot of 0.4 g of the fermentate obtained (fermented milk) was diluted by 2-fold with pure water, 20 µL of Carrez's Reagent I (53.5% (w/v) zinc sulfate) was added thereto and the mixture was vortexed, and 20 µl of Carrez's Reagent II (17.2% (w/v) potassium ferrocyanide) was added thereto and the mixture was vortexed. The resulting mixture was centrifuged at 4°C, 20620 g for 10 minutes, and the supernatant was filtered through a 0.22 µm filter and the filtrate was analyzed under the conditions shown in Table 2.

### [Table 2]

**Table 2: Organic acid analysis conditions**

| Analytical instrument | Shimadzu Corporation, High performance liquid chromatograph LC20 system |
|---|---|
| Column | Polymer column for organic acid analysis Tokyo Kasei Kogyo, TRANSGENOMIC Inc. ICSep ICE-ORH-801 6.5 mm I.D. × 300 mm, two columns connected |
| Guard column | ICSep ICE ORH-801 4.0 mm I.D. × 20 mm |
| Mobile phase | 7.5 mM p-toluenesulfonate |
| Reaction solution | 7.5 mM p-toluenesulfonate/150µM EDTA (2NA)/30 mM Bis Tris |
| Flow rate | 0.5 ml/min |
| Oven temperature | 55°C |
| Detector | Shimadzu Corporation, Electrical Conductivity detector CDD-10A |
| Injection amount | 10 µl |

The obtained results are shown in Tables 3 to 5. From Tables 3 and 4, fermentation promoting effects of fumaric acid in 0.01 to 2.5 mM and malic acid in 0.01 to 50 mM were confirmed in the case of using L. bulgaricus 2038. In addition, both malic acid and fumaric acid showed a fermentation promoting effect even at a low concentration of 0.01 mM. The results of measuring the organic acid concentrations after fermentation showed decrease in the concentrations of added malic acid and fumaric acid, and increase in the concentration of succinic acid (Table 5). That is, it was suggested that the added malic acid and fumaric acid were metabolized in the direction of malic acid to fumaric acid to succinic acid by the reductive TCA cycle.

### [Table 3]

**Table 3: Fermentation promoting effect of fumaric acid on L. delbrueckii subsp. bulgaricus 2038**

| | | Fermentation time | Reduction in fermentation time |
|---|---|---|---|
| No addition | | 7:25 | - |
| Fumaric acid added | 0.01 mM | 7:10 | 0:15 |
| | 0.05 mM | 7:05 | 0:20 |
| | 0.1 mM | 6:30 | 0:55 |
| | 0.5 mM | 6:00 | 1:25 |
| | 1mM | 6:25 | 1:00 |
| | 2.5 mM | 6:35 | 0:50 |

### [Table 4]

**Table 4: Fermentation promoting effect of malic acid on L. delbrueckii subsp. bulgaricus 2038**

| | | Fermentation time | Reduction in fermentation time |
|---|---|---|---|
| No addition | | 7:25 | - |
| Malic acid added | 0.01 mM | 7:10 | 0:15 |
| | 0.05 mM | 7:00 | 0:25 |
| | 0.1 mM | 6:40 | 0:45 |
| | 0.5 mM | 6:30 | 0:55 |
| | 1 mM | 6:25 | 1:00 |
| | 5 mM | 6:25 | 1:00 |
| | 10 mM | 6:35 | 0:50 |
| | 50 mM | 7:10 | 0:15 |

### [Table 5]

**Table 5: Concentrations of malic acid, fumaric acid, and succinic acid after fermentation by L. delbrueckii subsp. bulgaricus 2038**

| | | | Concentration of malic acid (mM) | Concentration of fumaric acid (mM) | Concentration of succinic acid (mM) |
|---|---|---|---|---|---|
| | Before fermentation (no addition) | | 0.15 | 0.00 | 0.08 |
| After fermentation | No addition | | 0.00 | 0.00 | 0.68 |
| | Malic acid added | 1 mM | 0.00 | 0.00 | 1.94 |
| | | 10 mM | 4.27 | 0.03 | 5.78 |
| | | 50 mM | 42.05 | 0.21 | 9.84 |
| | Fumaric acid added | 1 mM | 0.00 | 0.00 | 1.92 |

### Test Example 2-2: Amount of succinic acid production by L. delbrueckii subsp. bulgaricus with addition of malic acid and fumaric acid

The strains shown in Tables 6 and 7 were precultured in the preculture medium and then used. The activation medium used was a 10 mass% reduced skim milk medium containing 0.1 mass% yeast extract, sterilized at 121°C for 7 minutes. Here, the 10 mass% reduced skim milk medium is a 10 mass% aqueous solution of skim milk powder (fat 1 mass%, protein 34 mass%, lactose 54 mass%, ash 8 mass%, non-fat milk solid 96 mass%) (manufactured by Meiji Co., Ltd.) (in the above medium, lactose 5.4 mass%, non-fat milk solid 9.6 mass%). Frozen bacteria were added to the above-mentioned activation medium in an amount of 0.1 mass% (with respect to the amount of the activation medium) and statically cultured at 37°C for 16 hours to obtain an activation solution. An aliquot of 0.1 mass% of the activation solution obtained (with respect to the amount of activation medium) was added to another activation medium, and the mixture was allowed to stand at 37°C for 16 hours to obtain a lactic acid bacterial starter.

Then, fermentation was carried out using a fermentation medium as raw material milk. The fermentation medium was prepared by adding formic acid to the 10 mass% reduced skim milk medium so as to give a final concentration of 1 mM, and then sterilizing the mixture at 95°C, and was used. Further, an aqueous fumaric acid solution or an aqueous malic acid solution, adjusted to a pH of about 6.5 using NaOH before the sterilization, was added to the fermentation medium so as to give a final concentration of 1 mM. The pH of the fermentation medium was about 6.4. The lactic acid bacterial starter of each strain obtained above was inoculated into the fermentation medium in an amount of 2.5 mass% (with respect to the amount of the fermentation medium), statically cultured at 40°C, and fermented. The fermentation time was the time required for the medium to reach pH 4.6. The pH value was measured using a commercially available pH meter. The results obtained are shown in Tables 6 and 7. From Table 6, the addition of malic acid or fumaric acid enabled an increase in the concentration of succinic acid for all L. delbrueckii subsp. bulgaricus strains. Further, from Table 7, as in Test Example 1, the addition of malic acid or fumaric acid promoted fermentation in all 7 L. bulgaricus strains.

### [Table 6]

**Table 6: Concentration of organic acid in fermentate at completion of fermentation (pH 4.6)**

| | | Concentration (mM) | | |
|---|---|---|---|---|
| Lactobacillus delbrueckii subsp. bulgaricus | Additive | Fumaric acid | Malic acid | Succinic acid |
| No addition | None | ----- | 0.11 | 0.07 |
| No addition | Fumaric acid | 1.11 | 0.11 | 0.07 |
| No addition | Malic acid | ----- | 0.99 | 0.08 |
| 2038 | None Fumaric acid Malic acid | ----- | ----- | 0.71 |
| | | ----- | ----- | 1.67 |
| | | ----- | ----- | 1.71 |
| OLL1255 | None Fumaric acid Malic acid | ----- | ----- | 0.72 |
| | | 0.15 | ----- | 1.49 |
| | | ----- | 0.35 | 1.03 |
| P1902901 | None Fumaric acid Malic acid | ----- | ----- | 0.72 |
| | | ----- | ----- | 1.59 |
| | | ----- | ----- | 1.59 |
| OLL1171 | None Fumaric acid Malic acid | ----- | ----- | 0.61 |
| | | ----- | ----- | 1.58 |
| | | ----- | ----- | 1.57 |
| OLL205013 | None Fumaric acid Malic acid | ----- | ----- | 0.38 |
| | | ----- | ----- | 1.54 |
| | | ----- | ----- | 1.55 |
| OLL1 073R-1 | None Fumaric acid Malic acid | ----- | ----- | 0.67 |
| | | ----- | ----- | 1.73 |
| | | ----- | ----- | 1.67 |
| OLL1247 | None Fumaric acid Malic acid | ----- | ----- | 0.67 |
| | | ----- | ----- | 1.72 |
| | | ----- | ----- | 1.67 |

### [Table 7]

**Table 7: Fermentation promoting effect of malic acid and fumaric acid on L. delbrueckii subsp. bulgaricus**

| Lactobacillus delbrueckii subsp. bulgaricus | No addition | Malic acid (1 mM) added | Fumaric acid (1 mM) added | Reduction in fermentation time by addition of malic acid (1 mM) | Reduction in fermentation time by addition of fumaric acid (1 mM) |
|---|---|---|---|---|---|
| 2038 | 4:33 | 3:20 | 3:21 | 1:13 | 1:12 |
| OLL1255 | 7:42 | 3:17 | 3:05 | 4:25 | 4:37 |
| P1902901 | 5:48 | 3:56 | 4:09 | 1:52 | 1:39 |
| OLL1171 | 7:55 (pH4.79) | 4:32 | 4:57 | >3:00 | >3:00 |
| OLL205013 | 7:56 (pH5.48) | 6:38 | 6:30 | >1:30 | >1:30 |
| OLL1073R-1 | 4:36 | 3:29 | 3:46 | 1:07 | 0:50 |
| OLL1247 | 8:00 (pH4.75) | 3:28 | 3:38 | >4:30 | >4:30 |

### Test Example 3: Examination of fermentation promoting effect of fumaric acid on lactic acid bacterial species other than L. delbrueckii subsp. bulgaricus

First, type strains of the following strains other than Lactobacillus delbrueckii subsp. bulgaricus were prepared.
(8) Lactobacillus acidophilus JCM 1132T
(9) Lactobacillus gasseri JCM 1131T
(10) Lactobacillus rhamnosus JCM 1136T
(11) Lactobacillus reuteri JCM 1112T
(12) Lactobacillus salivarius JCM 1231T
(13) Lactobacillus pentosus JCM 1558T

The above strains were used after precultured in an preculture medium. The activation medium used was a 10% reduced skim milk medium containing 0.1% yeast extract, sterilized at 121°C for 7 minutes. Frozen bacteria were added to the above-mentioned activation medium in an amount of 1% (with respect to the amount of the activation medium) and statically cultured at 37°C for 24 hours to obtain an activation solution. An aliquot of 1% of the activation solution obtained (with respect to the amount of activation medium) was added to another activation medium, and the mixture was allowed to stand at 37°C for 24 hours to obtain a lactic acid bacterial starter.

Then, fermentation was carried out using a fermentation medium (raw material milk). The fermentation medium was prepared by adding formic acid to the 10% reduced skim milk medium so as to give a final concentration of 1 mM, and then sterilizing the mixture at 95°C, and was used. Further, an aqueous fumaric acid solution, adjusted to a pH of about 6.5 using NaOH before the sterilization, was added to the fermentation medium so as to give a final concentration of 1 mM. The lactic acid bacterial starter precultured as above of each strain was inoculated into the fermentation medium in an amount of 1% (with respect to the amount of the fermentation medium), statically cultured at 37°C, and fermented. The fermentation time was the time required for the medium to reach pH 4.6. The results obtained are shown in Table 8. The addition of fumaric acid promoted fermentation by L. acidophilus, L. gasseri, L. rhamnosus, L. reuteri, L. salivarius, and L. pentosus.

### [Table 8]

**Table 8: Fermentation promoting effect of fumaric acid**

| | | Fermentation time (Time to reach pH 4.6) | |
|---|---|---|---|
| Bacterial species | Strain | No addition | Fumaric acid (1 mM) added |
| L. acidophilus | JCM 1132T | 16:50 | 15:45 |
| L. gasseri | JCM 1131T | >24 | 24:00 |
| L. rhamnosus | JCM 1136T | >24 | 23:20 |
| L. reuteri | JCM 1112T | >24 | 13:20 |
| L. salivarius | JCM 1231T | >24 | 13:30 |
| L. pentosus | JCM 1558T | >24 | 14:50 |

### Test Example 4: Examination of fermentation promoting effect of malic acid on lactic acid bacterial species other than L. delbrueckii subsp. bulgaricus

First, the type strains of the following bacterial species were prepared.
(12) Lactobacillus salivarius JCM 1231T
(13) Lactobacillus pentosus JCM 1558T
(14) Lactobacillus kefiranofaciens subsp. kefirgranum JCM 8572T

The procedure was carried out in the same manner as in Test Example 3 except that malic acid was added instead of fumaric acid. The results obtained are shown in Table 9. The addition of malic acid promoted fermentation by L. salivarius, L. pentosus, and Lactobacillus kefiranofaciens subsp. kefirgranum.

### [Table 9]

**Table 9: Fermentation promoting effect of malic acid**

| | | Fermentation time (Time to reach pH 4.6) | |
|---|---|---|---|
| Bacterial species | Strain | No addition | Malic acid (1 mM) added |
| L. salivarius | JCM 1231T | >24 | 14:40 |
| L. pentosus | JCM 1558T | >24 | 11:45 |
| L. kefiranofaciens subsp. kefirgranum | JCM 8572T | >24 | 13:20 |

### Test Example 5: Measurement of concentrations of malic acid, fumaric acid, and succinic acid in L. rhamnosus and L. salivarius

The measurement of the organic acid concentration after the completion of fermentation by L. rhamnosus and L. salivarius in Test Example 3 or 4 was carried out in the same manner as in Test Example 2-1. The results obtained are shown in Table 10. The addition of malic acid or fumaric acid promoted production of succinic acid by L. rhamnosus and L. salivarius.

### [Table 10]

**Table 10: Concentrations of malic acid, fumaric acid, and succinic acid after fermentation by L. rhamnosus and L. salivarius**

| | | Concentration of malic acid (mM) | Concentration of fumaric acid (mM) | Concentration of succinic acid (mM) | Fermentation time |
|---|---|---|---|---|---|
| L. rhamnosus | No addition | 0.16 | 0.04 | 0.10 | >24 hr |
| | Fumaric acid (1 mM) added | - | - | 1.39 | 23:20 |
| L. salivarius | No addition | 0.08 | - | 0.10 | >24 hr |
| | Malic acid (1 mM) added | 0.75 | 0.35 | 0.28 | 14:40 |
| | Fumaric acid (1 mM) added | 0.51 | 0.62 | 0.29 | 13:30 |

### Test Example 6: Examination of fermentation promoting effect of fumaric acid on Lactobacillus delbrueckii subsp. bulgaricus with addition of inosinic acid

Frozen bacteria of the following Lactobacillus delbrueckii subsp. bulgaricus strains were prepared.
(1) Lactobacillus delbrueckii subsp. bulgaricus 2038
(2) Lactobacillus delbrueckii subsp. bulgaricus OLL1073R-1
(3) Lactobacillus delbrueckii subsp. bulgaricus P1902901
(4) Lactobacillus delbrueckii subsp. bulgaricus OLL1171
(5) Lactobacillus delbrueckii subsp. bulgaricus OLL1255
(6) Lactobacillus delbrueckii subsp. bulgaricus OLL1247
(7) Lactobacillus delbrueckii subsp. bulgaricus OLL205013

The above strains were used after precultured in an preculture medium. The activation medium used was a 10% reduced skim milk medium containing 0.1% yeast extract, sterilized at 121°C for 7 minutes. Frozen bacteria were added to the above-mentioned activation medium in an amount of 1% (with respect to the amount of the activation medium) and statically cultured at 37°C for 24 hours to obtain an activation solution. An aliquot of 1% of the activation solution obtained (with respect to the amount of activation medium) was added to another activation medium, and the mixture was allowed to stand at 37°C for 24 hours to obtain a lactic acid bacterial starter.

Then, fermentation was carried out using a fermentation medium (raw material milk). The fermentation medium was prepared by adding inosinic acid to the 10% reduced skim milk medium so as to give a final concentration of 1 mM, and then sterilizing the mixture at 95°C, and was used. Further, an aqueous fumaric acid solution, adjusted to a pH of about 6.5 using NaOH before the sterilization, was added to the fermentation medium so as to give a final concentration of 1 mM. The lactic acid bacterial starter precultured as above of each strain was inoculated into the fermentation medium in an amount of 1% (with respect to the amount of the fermentation medium), statically cultured at 37°C, and fermented. The fermentation time was the time required for the medium to reach pH 4.6. The pH value was measured using a pH meter. The results obtained are shown in Table 11. From Table 11, the addition of fumaric acid promoted fermentation in all 7 L. bulgaricus strains.

### [Table 11]

**Table 11: Fermentation promoting effect of fumaric acid on L. delbrueckii subsp. bulgaricus with addition of inosinic acid**

| | Fermentation time (Time to reach pH 4.6) | | Reduction in fermentation time |
|---|---|---|---|
| Strain | No addition | Fumaric acid (1 mM) added | With addition of fumaric acid (1 mM) |
| 2038 | 4:45 | 4:20 | 0:25 |
| OLL1 073R-1 | 5:20 | 4:45 | 0:35 |
| P1902901 | 5:55 | 5:30 | 0:25 |
| OLL1171 | 6:25 | 6:00 | 0:25 |
| OLL1255 | 5:35 | 5:15 | 0:20 |
| OLL1247 | 4:35 | 4:15 | 0:20 |
| OLL205013 | 9:25 | 8:45 | 0:40 |

### Test Example 7: Examination of fermentation promoting effect of malic acid on L. delbrueckii subsp. bulgaricus with addition of inosinic acid

Frozen bacteria of the following Lactobacillus delbrueckii subsp. bulgaricus strains were prepared.
(1) Lactobacillus delbrueckii subsp. bulgaricus 2038
(2) Lactobacillus delbrueckii subsp. bulgaricus OLL1073R-1
(3) Lactobacillus delbrueckii subsp. bulgaricus P1902901
(4) Lactobacillus delbrueckii subsp. bulgaricus OLL1171
(5) Lactobacillus delbrueckii subsp. bulgaricus OLL1247

The procedure was carried out in the same manner as in Test Example 6 except that malic acid was added instead of fumaric acid. The results obtained are shown in Table 12. The addition of malic acid promoted fermentation by L. bulgaricus 2038, OLL1073R-1, P1902901, OLL1171, and OLL1247.

### [Table 12]

**Table 12: Fermentation promoting effect of malic acid on L. delbrueckii subsp. bulgaricus with addition of inosinic acid**

| | Fermentation time (Time to reach pH 4.6) | | Reduction in fermentation time |
|---|---|---|---|
| Strain | No addition | Malic acid (1 mM) added | With addition of malic acid (1 mM) |
| 2038 | 4:45 | 4:30 | 0:15 |
| OLL1073R-1 | 5:20 | 4:50 | 0:30 |
| P1902901 | 5:55 | 5:25 | 0:30 |
| OLL1171 | 6:25 | 5:50 | 0:35 |
| OLL1247 | 4:35 | 4:15 | 0:20 |

### Test Example 8: Effect of malic acid or fumaric acid on succinic acid production by L. delbrueckii subsp. of bulgaricus with addition of inosinic acid

The strains shown in Tables 13 and 14 were precultured in the preculture medium and then used. The activation medium used was a 10 mass% reduced skim milk medium containing 0.1 mass% yeast extract, sterilized at 121°C for 7 minutes. Here, the 10 mass% reduced skim milk medium is a 10 mass% aqueous solution of skim milk powder (fat 1 mass%, protein 34 mass%, lactose 54 mass%, ash 8 mass%, non-fat milk solid 96 mass%) (manufactured by Meiji Co., Ltd.) (in the above medium, lactose 5.4 mass%, non-fat milk solid 9.6 mass%). Frozen bacteria were added to the above-mentioned activation medium in an amount of 0.1 mass% (with respect to the amount of the activation medium) and statically cultured at 37°C for 16 hours to obtain an activation solution. An aliquot of 0.1 mass% of the activation solution obtained (with respect to the amount of activation medium) was added to another activation medium, and the mixture was allowed to stand at 37°C for 16 hours to obtain a lactic acid bacterial starter.

Then, fermentation was carried out using a fermentation medium as raw material milk. The fermentation medium was prepared by adding inosinic acid to the 10 mass% reduced skim milk medium so as to give a final concentration of 1 mM, and then sterilizing the mixture at 95°C, and was used. Further, an aqueous fumaric acid solution or an aqueous malic acid solution, adjusted to a pH of about 6.5 using NaOH before the sterilization, was added to the fermentation medium so as to give a final concentration of 1 mM. The pH of the fermentation medium was about 6.4. The lactic acid bacterial starter of each strain obtained above was inoculated into the fermentation medium in an amount of 2.5 mass% (with respect to the amount of the fermentation medium), statically cultured at 40°C, and fermented. The fermentation time was the time required for the medium to reach pH 4.6. The pH value was measured using a commercially available pH meter. The results obtained are shown in Tables 13 and 14. From Table 13, in the medium containing inosinic acid, the addition of malic acid or fumaric acid enabled an increase in the concentration of succinic acid for all L. delbrueckii subsp. bulgaricus strains. Therefore, in the inosinic acid-added medium as well as in the formic acid-added medium, the addition of malic acid or fumaric acid was shown to enable an increase in produced amount of succinic acid by L. delbrueckii subsp. bulgaricus. Further, from Table 14, as in Test Example 6, the addition of malic acid or fumaric acid promoted fermentation in all 7 L. bulgaricus strains.

### [Table 13]

**Table 13: Concentration of organic acid in fermentate at completion of fermentation (pH 4.6)**

| | | Concentration (mM) | | |
|---|---|---|---|---|
| Lactobacillus delbrueckii subsp. bulgaricus | Additive | Fumaric acid | Malic acid | Succinic acid |
| No addition | None | ----- | 0.14 | 0.06 |
| No addition | Fumaric acid | 1.12 | 0.13 | 0.06 |
| No addition | Malic acid | ----- | 1.06 | 0.06 |
| 2038 | None Fumaric acid Malic acid | ----- | ----- | 0.55 |
| | | ----- | ----- | 1.43 |
| | | ----- | ----- | 1.40 |
| OLL1255 | None Fumaric acid Malic acid | ----- | ----- | 0.60 |
| | | ----- | ---- | 1.49 |
| | | ----- | ----- | 1.46 |
| P1902901 | None Fumaric acid Malic acid | ----- | ----- | 0.48 |
| | | ----- | ----- | 1.40 |
| | | ----- | ----- | 1.38 |
| OLL1171 | None Fumaric acid Malic acid | ----- | ----- | 0.55 |
| | | ----- | ----- | 1.43 |
| | | ----- | ----- | 1.40 |
| OLL205013 | None Fumaric acid Malic acid | ----- | ----- | 0.62 |
| | | ----- | ----- | 1.60 |
| | | ----- | ----- | 1.53 |
| OLL1 073R-1 | None Fumaric acid Malic acid | ----- | ----- | 0.59 |
| | | ----- | ----- | 1.52 |
| | | ---- | ----- | 1.50 |
| OLL1247 | None Fumaric acid Malic acid | ----- | ----- | 0.62 |
| | | ----- | ----- | 1.57 |
| | | ----- | ----- | 1.53 |

### [Table 14]

**Table 14: Fermentation promoting effect of malic acid and fumaric acid on L. delbrueckii subsp. bulgaricus**

| Lactobacillus delbrueckii subsp. bulgaricus | No addition | Fumaric acid (1 mM) added | Malic acid (1 mM) added | Reduction in fermentation time by addition of fumaric acid (1 mM) | Reduction in fermentation time by addition of malic acid (1 mM) |
|---|---|---|---|---|---|
| 2038 | 3:51 | 3:13 | 3:15 | 0:38 | 0:36 |
| OLL1255 | 3:29 | 3:10 | 3:14 | 0:19 | 0:15 |
| P1902901 | 4:26 | 4:00 | 3:55 | 0:26 | 0:31 |
| OLL1171 | 4:13 | 3:28 | 3:27 | 0:45 | 0:46 |
| OLL205013 | 7:40 | 7:30 | 6:50 | 0:10 | 0:50 |
| OLL1073R-1 | 3:35 | 2:58 | 3:00 | 0:37 | 0:35 |
| OLL1247 | 3:24 | 3:09 | 3:06 | 0:15 | 0:18 |

### Test Example 9: Effect of malic acid or fumaric acid on proliferation property and metabolite production of L. delbrueckii subsp. of bulaaricus

Frozen bacteria of Lactobacillus delbrueckii subsp. bulgaricus OLL1073R-1 were prepared.

The composition of the medium was as shown in Table 15 below. Raw material components other than anhydrous crystalline glucose were dissolved in Elix water, the mixture was adjusted to pH 6.65 using sodium hydroxide, and then diluted in measuring flask to 70 wt% of the charge amount of the medium. Anhydrous crystalline glucose was dissolved in Elix water, and diluted in measuring flask to 30 wt% of the charge amount of the medium. When malic acid or fumaric acid was added to the medium, malic acid or fumaric acid was dissolved in Elix water so as to give a final concentration of 4 mM together with raw material components other than anhydrous crystalline glucose and the resulting mixture was added to the medium.

In addition, the raw material components other than sugar solution and sugar were sterilized separately by heating at 110°C for 1 minute in an autoclave and then aseptically mixed and used.

### [Table 15]

**Table 15: Composition of culture medium**

| | Blending ratio %(w/w) |
|---|---|
| Skim milk powder | 4 |
| Anhydrous crystalline glucose | 7 |
| Beer yeast extract Meast P2G | 0.5 |
| Fish meat extract Bacterio-N-KN | 1 |
| Emulsifier Sunsoft Q17S | 0.05 |
| Antifoaming agent Awabreak L-01 | 0.1 |
| Water | balance |
| Total | 100 |

An aliquot of 1.5 kg of the above medium was adjusted to pH 5.4 using 6 N potassium carbonate, to the medium, 0.30 mass% of frozen bacteria (with respect to the amount of medium) was inoculated, and the bacteria were cultured at 37°C while top ventilating with nitrogen at a flow rate of 0.5 L/min and stirring under the condition of 150 rpm.

Fig. 1 shows the change of EPS concentration with the culturing time.

As shown in Fig. 1, in the case where malic acid or fumaric acid was added, EPS was confirmed to exhibit higher concentration compared with the control. Therefore, the addition of malic acid or fumaric acid was found to promote the production of EPS, which is a metabolite of L. delbrueckii subsp. bulgaricus. In addition, since the addition of malic acid or fumaric acid enables an increase in EPS concentration in a shorter time as compared with the control, malic acid or fumaric acid was reconfirmed to promote the fermentation by L. delbrueckii subsp. bulgaricus.

The EPS concentration was measured under the following conditions.

To an aliquot of the culture solution weighed at 1.5 g, MilliQ water was added up to a total weight of 10 g. After adding 100% trichloroacetic acid, the resultant mixture was mixed well, centrifuged (4°C, 13400 g, 10 min), and the supernatant was recovered. A 5 ml aliquot of a 10% trichloroacetic acid solution was added to the precipitate to form a suspension, and then the suspension was centrifuged again to recover the supernatant. To the supernatant, cooled 99.5% ethanol was added at twice the amount of the supernatant, the mixture was mixed by inversion, and then allowed to stand at -20°C overnight.

The supernatant was removed by centrifuging (4°C, 13400 g, 20 min), and 20 ml of cooled 66% ethanol was added thereto to suspend the precipitate. The supernatant was removed by centrifuging (4°C, 13400 g, 20 min), the centrifugate was air-dried to remove ethanol, and MilliQ water was added thereto to a fixed volume of 10 ml, which was used as an analysis sample. The EPS concentration of the analysis sample was measured by the phenolsulfuric acid method.

| | | |
|---|---|---|
| 0-1 | Form PCT/RO/134 | JPO-PAS i410 |
| 0-1-1 | The indications to this deposited microorganism or other biological material (PCT Rule 13-2) are prepared as shown in the right column. | |
| 0-2 | International application number | |
| 0-3 | Applicant's or agent's file reference | 235003 |

| | | |
|---|---|---|
| 1 | The indications below relate to the microorganisms or biological materials described in the detailed description of the invention. | 0043 |
| 1-1 | Paragraph number | |
| 1-3 | Identification of the deposit | International Patent Organism Depositary, National Institute of Technology and Evaluation |
| 1-3-1 | Name of depositary institution | |
| 1-3-2 | Address of depositary institution | 2-5-8 Kazusakamatari, Kisarazu-shi, Chiba-ken 292-0818, JAPAN |
| 1-3-3 | Date of deposit | February 22, 1999 (22.02.1999) |
| 1-3-4 | Accession number | IPOD FERM BP-10741 |
| 1-5 | Designated states for which indications are made | All designated states |
| 2 | The indications below relate to the microorganisms or biological materials described in the detailed description of the invention. Paragraph number | 0045 |
| 2-1 | | |
| 2-3 2-3-1 | Identification of the deposit Name of depositary institution | International Patent Organism Depositary, National Institute of Technology and Evaluation |
| 2-3-2 | Address of depositary institution | 2-5-8 Kazusakamatari, Kisarazu-shi, Chiba-ken 292-0818, JAPAN |
| 2-3-3 | Date of deposit | March 13, 2013 (13.03.2013) |
| 2-3-4 | Accession number | IPOD NITE BP-01569 |
| 2-5 | Designated states for which indications are made | All designated states |
| 3 | The indications below relate to the microorganisms or biological materials described in the detailed description of the invention. | 0046 |
| 3-1 | Paragraph number | |
| 3-3 3-3-1 | Identification of the deposit Name of depositary institution | International Patent Organism Depositary, National Institute of Technology and Evaluation |
| 3-3-2 | Address of depositary institution | 2-5-8 Kazusakamatari, Kisarazu-shi, Chiba-ken 292-0818, JAPAN |
| 3-3-3 | Date of deposit | February 10, 2005 (10.02.2005) |
| 3-3-4 | Accession number | IPOD NITE BP-76 |
| 3-5 | Designated states for which indications are made | All designated states |
| 4 | The indications below relate to the microorganisms or biological materials described in the detailed description of the invention. Paragraph number | 0047 |
| 4-1 | | |
| 4-3 4-3-1 | Identification of the deposit Name of depositary institution | International Patent Organism Depositary, National Institute of Technology and Evaluation |
| 4-3-2 | Address of depositary institution | 2-5-8 Kazusakamatari, Kisarazu-shi, Chiba-ken 292-0818, JAPAN |
| 4-3-3 | Date of deposit | March 06, 2014 (06.03.2014) |
| 4-3-4 | Accession number | IPOD NITE BP-01814 |
| 4-5 | Designated states for which indications are made | All designated states |
| 5 | The indications below relate to the microorganisms or biological materials described in the detailed description of the invention. Paragraph number | 0048 |
| 5-1 | | |
| 5-3 5-3-1 | Identification of the deposit Name of depositary institution | International Patent Organism Depositary, National Institute of Technology and Evaluation |
| 5-3-2 | Address of depositary institution | 2-5-8 Kazusakamatari, Kisarazu-shi, Chiba-ken 292-0818, JAPAN |
| 5-3-3 | Date of deposit | February 03, 2017 |
| 5-3-4 | Accession number | (03.02.2017) IPOD NITE BP-02411 |
| 5-5 | Designated states for which indications are made | All designated states |

### For receiving Office use only

| | | |
|---|---|---|
| 0-4 | This sheet was received with the international application (Yes, No) | |
| 0-4-1 | Authorized officer | |

### For International Bureau use only

| | | |
|---|---|---|
| 0-5 | This sheet was received by the International Bureau on: | |
| 0-5-1 | Authorized officer | |

## Claims

1. A lactic acid bacterial fermentation promoter, comprising at least one organic acid selected from malic acid and fumaric acid.

2. The fermentation promoter according to claim 1, wherein the fermentation promoter is for use in combination with a nucleic acid raw material.

3. The fermentation promoter according to claim 2, wherein the nucleic acid raw material is at least one selected from formic acid and a compound having a purine skeleton with a hydrogen atom attached to a carbon atom at 2-position.

4. The fermentation promoter according to any one of claims 1 to 3, wherein the lactic acid bacteria comprises genus Lactobacillus.

5. The fermentation promoter according to claim 4, wherein the genus Lactobacillus is at least one selected from the group consisting of Lactobacillus delbrueckii, Lactobacillus delbrueckii subsp. bulgaricus, Lactobacillus acidophilus, Lactobacillus gasseri, Lactobacillus rhamnosus, Lactobacillus reuteri, Lactobacillus salivarius, and Lactobacillus pentosus.

6. A lactic acid bacterial starter comprising lactic acid bacteria and at least one organic acid selected from malic acid and fumaric acid.

7. The lactic acid bacterial starter according to claim 6, further comprising a nucleic acid raw material.

8. A method for producing fermented milk, comprising: performing lactic acid bacterial fermentation in the presence of at least one organic acid selected from malic acid and fumaric acid.

9. The method according to claim 8, further comprising performing lactic acid bacterial fermentation in the presence of the organic acid and a nucleic acid raw material.

10. The method according to claim 8 or 9, wherein the fermented milk comprises succinic acid.

11. The method according to claim 10, wherein the succinic acid is an endogenous organic acid produced by the lactic acid bacteria.

12. A fermented milk obtained by the method according to any one of claims 8 to 11,
the fermented milk comprising lactic acid bacteria and succinic acid.

13. The fermented milk according to claim 12, wherein the succinic acid is an endogenous organic acid produced by the lactic acid bacteria.

14. The fermented milk according to claim 13, wherein the content of the succinic acid is 0.15 mM or more with respect to the total amount of fermented milk.

15. The fermented milk according to any one of claims 12 to 14, further comprising at least one organic acid selected from malic acid and fumaric acid.

16. A method for promoting lactic acid bacterial fermentation, comprising: performing lactic acid bacterial fermentation in the presence of at least one organic acid selected from malic acid and fumaric acid.

17. The method for promoting lactic acid bacterial fermentation according to claim 16, further comprising a nucleic acid law material.

18. A method for producing a lactic acid bacterial starter, comprising: culturing lactic acid bacteria in the presence of at least one organic acid selected from malic acid and fumaric acid.

19. The method according to claim 18, further comprising culturing lactic acid bacteria in the presence of the organic acid and a nucleic acid raw material.

20. The method according to claim 18 or 19, wherein the lactic acid bacterial starter comprises lactic acid bacteria and at least one organic acid selected from malic acid and fumaric acid.

21. The method according to claim 20, wherein the lactic acid bacterial starter further comprises succinic acid.

22. The method according to claim 21, wherein the succinic acid is an endogenous organic acid produced by the lactic acid bacteria.

23. A fermented milk having a succinic acid at a content of 0.15 mM or more with respect to the total amount of fermented milk.

24. The fermented milk according to claim 23, further comprising at least one organic acid selected from malic acid and fumaric acid.

25. The fermented milk according to claim 23 or 24, wherein the succinic acid is an endogenous organic acid produced by the lactic acid bacteria.

26. A method for producing a lactic acid bacterial fermentation metabolite, comprising: performing lactic acid bacterial fermentation in the presence of at least one organic acid selected from malic acid and fumaric acid.
